(19) **Europäisches Patentamt / European Patent Office / Office européen des brevets**

(11) **EP 4 386 001 B1**

(12) **EUROPEAN PATENT SPECIFICATION**

(45) Date of publication and mention
of the grant of the patent:
**04.06.2025 Bulletin 2025/23**

(21) Application number: **23216444.2**

(22) Date of filing: **13.12.2023**

(51) International Patent Classification (IPC):
***G01N 33/49*** *(2006.01)*       ***G01N 33/72*** *(2006.01)*
***C07K 16/18*** *(2006.01)*       ***G01N 30/88*** *(2006.01)*

(52) Cooperative Patent Classification (CPC):
**G01N 33/49; G01N 33/723;** G01N 2030/8822

(54) **METHOD FOR ESTIMATING HEMOGLOBIN A1C LEVEL, INFORMATION PROCESSING APPARATUS, AND COMPUTER-READABLE STORAGE MEDIUM**

VERFAHREN ZUR SCHÄTZUNG DES HÄMOGLOBIN-A1C-SPIEGELS, INFORMATIONSVERARBEITUNGSVORRICHTUNG UND COMPUTERLESBARES SPEICHERMEDIUM

PROCÉDÉ D'ESTIMATION DU NIVEAU D'HÉMOGLOBINE A1C, APPAREIL DE TRAITEMENT D'INFORMATIONS ET SUPPORT D'INFORMATIONS LISIBLE PAR ORDINATEUR

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **16.12.2022 JP 2022201260**

(43) Date of publication of application:
**19.06.2024 Bulletin 2024/25**

(73) Proprietor: **ARKRAY, Inc.**
**Kyoto-shi, Kyoto 601-8045 (JP)**

(72) Inventor: **ISHIKAWA, Kazuki**
**Kyoto, 602-0008 (JP)**

(74) Representative: **Dehns**
**10 Old Bailey**
**London EC4M 7NG (GB)**

(56) References cited:
**WO-A1-2020/067396       CN-A- 109 073 616**
**JP-A- 2001 221 788       JP-A- 2016 183 871**
**JP-A- 2017 194 349**

**EP 4 386 001 B1**

**Description**

BACKGROUND

Technical Field

The present invention relates to a method for estimating a hemoglobin A1c level, an information processing apparatus, and a computer-readable storage medium storing an estimation program.

Related Art

Japanese Patent Application Laid-Open (JP-A) No. 2012-215470 (Patent Literature 1) describes measuring the percentage (%) of stable hemoglobin A1c in a case where a peak derived from abnormal hemoglobin E is identified by calculating the area of the peak and correcting the peak area of the stable hemoglobin A1c or the peak areas of all hemoglobins using the calculation result.

In blood specimen containing mutant hemoglobin (hereinafter, "mutant Hb", also called variant hemoglobin), it is difficult to measure a stable hemoglobin A1c level (hereinafter, an "HbAlc level"), which is an indicator of the blood glucose level. For example, in a blood specimen containing mutant Hb, glycated mutant Hb may be measured, and, in an affinity method, an immunization method, or an enzyme method, glycated mutant Hb is simultaneously measured with HbA1c.

Specifically, in a blood specimen from a subject which is heterozygous for mutant Hb, the total level of HbA1c and glycated mutant Hb is measured. Furthermore, in a blood specimen which is homozygous for mutant Hb, the percentage of glycated mutant Hb is measured. However, the measured levels of glycated mutant Hb and HbA1c do not necessarily coincide with each other, and thus, the level measured using the blood specimen containing mutant Hb cannot be considered as an indicator of the blood glucose level.

SUMMARY

At least preferred embodiments of the present invention estimate an HbA1c level, which is an indicator of the blood glucose level, even with a blood specimen containing mutant Hb.

According to an aspect of the present invention, a method for estimating a hemoglobin A1c level includes acquiring in advance a correlation between a hemoglobin A1c level which is a value based on the ratio of the peak value of a hemoglobin A1c peak containing hemoglobin A1c to the total value of the peak values of peaks related to hemoglobin A and a glycated mutant hemoglobin level which is a value based on the ratio of the peak value of a glycated mutant hemoglobin peak to the total value of the peak value of a peak containing mutant hemoglobin and the peak value of the glycated mutant hemoglobin peak containing glycated mutant hemoglobin formed by glycating the mutant hemoglobin, from a chromatogram obtained by subjecting a blood specimen containing the hemoglobin A and the mutant hemoglobin that is collected from each of a plurality of subjects to liquid chromatography, deriving a glycated mutant hemoglobin level from a chromatogram obtained by subjecting a blood specimen which is a measurement target collected from a subject having the mutant hemoglobin to liquid chromatography, and estimating a hemoglobin A1c level based on the derived glycated mutant hemoglobin level and the correlation.

According to embodiments of the present invention, an HbA1c level, which is an indicator of the blood glucose level, can be estimated even with a blood specimen containing mutant Hb.

BRIEF DESCRIPTION OF THE DRAWINGS

Certain embodiments of the present invention will now be described by way of example only and with reference to the accompanying drawings, in which:

Fig. 1A is a diagram illustrating an example of a chromatogram of hemoglobins obtained by subjecting a blood specimen to liquid chromatography.
Fig. 1B is a diagram illustrating an example of a chromatogram of hemoglobins obtained by subjecting a blood specimen to liquid chromatography.
Fig. 1C is a diagram illustrating an example of a chromatogram of hemoglobins obtained by subjecting a blood specimen to liquid chromatography.
Fig. 2 is a diagram of a table showing components of eluents used when analyzing a blood specimen using liquid chromatography.
Fig. 3 is a diagram of a table showing glycated HbC levels and HbA1c levels derived from chromatograms of liquid chromatography.

Fig. 4 is a diagram of a graph showing a correlation between the glycated HbC level and the HbA1c level obtained from blood specimens.

Fig. 5 is a diagram of a table showing glycated HbC levels derived from chromatograms of liquid chromatography, HbA1c levels derived from a correlation formula, and known HbA1c levels.

Fig. 6 is a diagram of a graph showing a correlation between the HbA1c level derived from the correlation formula and the known HbA1c level.

Fig. 7 is a block diagram showing a hardware configuration of an exemplary information processing apparatus.

Fig. 8 is a block diagram showing a functional configuration of the exemplary information processing apparatus.

Fig. 9 is a flow diagram showing an exemplary flow of control by the information processing apparatus.

DETAILED DESCRIPTION

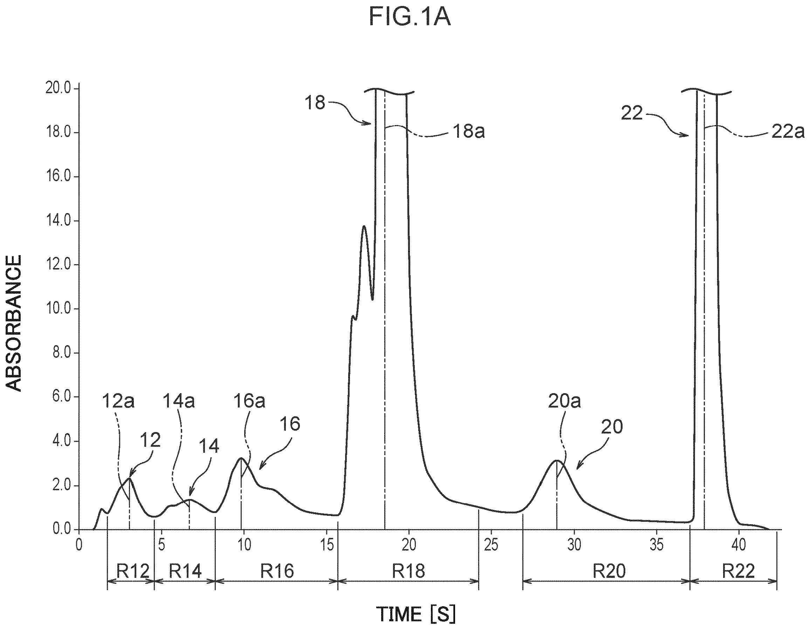

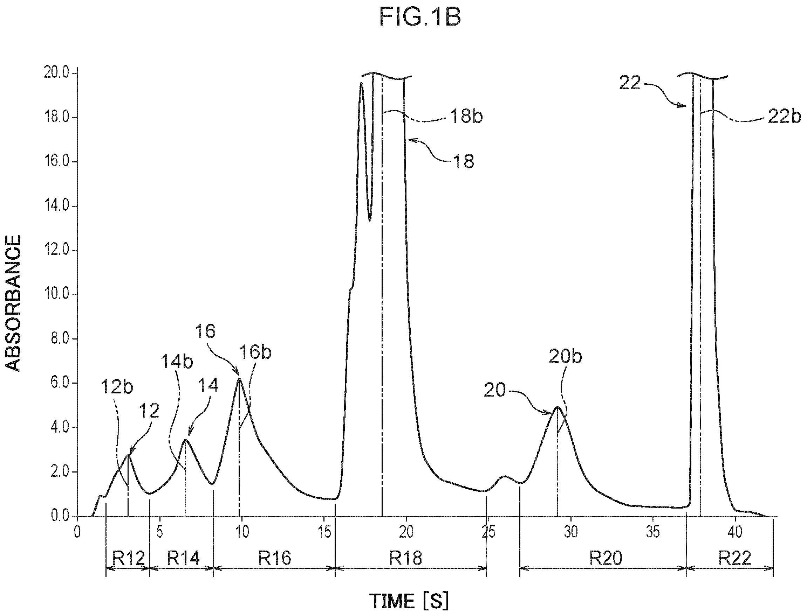

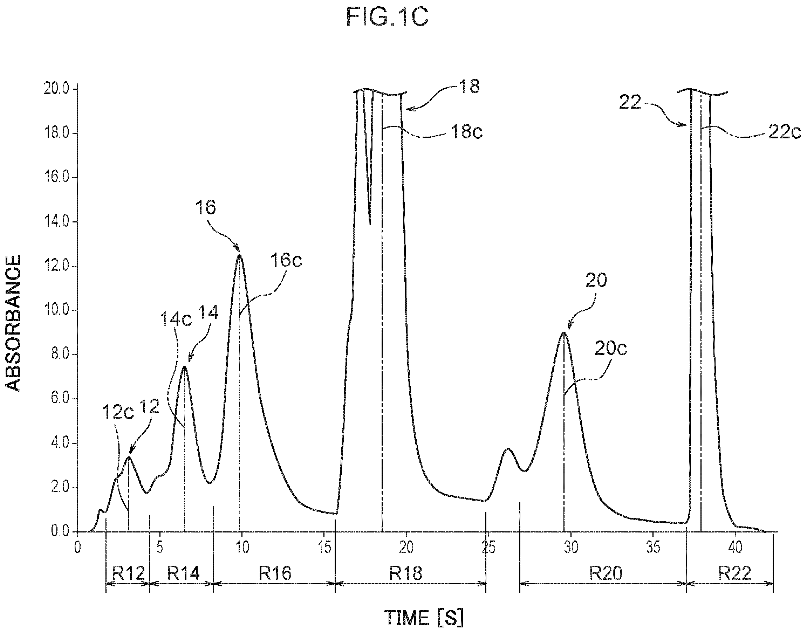

Embodiments of a method for estimating a hemoglobin A1c level, an information processing apparatus, and an estimation program of the present invention will be described using Figs 1 to 9. The same reference numerals in the drawings indicate the same parts even when a particular description is not provided. The "peak value" described herein is the height or area of each peak observed in a chromatogram, and it is possible to use a relative value or an absolute value. The relative value may be the ratio of the area of each peak to the entire area of the chromatogram, to the entire area of peaks related to hemoglobin in the chromatogram, or to the area of a specific peak (for example, an HbA0 peak).

A majority of hemoglobins in a healthy adult is HbA, and small amounts thereof are HbF and HbA2. Hemoglobin is a tetramer, with one example being HbA which consists of two $\alpha$ chains and two $\beta$ chains. When a mutation occurs in the gene sequence that controls the production of the $\alpha$ chain or $\beta$ chain, a chain of which the amino acid sequence is different from the normal amino acid sequence is produced, or the production of the $\alpha$ chain or $\beta$ chain is suppressed. As a result, a type of hemoglobin that is different from the normal hemoglobin is generated. Such type of hemoglobin is generally referred to as mutant hemoglobin. While the hemoglobin genotype of a healthy individual is HbA/HbA, which is homozygous, the genotype of an individual having a mutant hemoglobin is HbA/HbV, which is heterozygous (HbV is mutant hemoglobin), or HbV/HbV, which is homozygous (in a case where the types of HbV are different from each other, the genotype is heterozygous).

First, a blood specimen containing hemoglobin A and hemoglobin C (hereinafter referred to as "HbC") that is collected from each of a plurality of subjects is subjected to liquid chromatography. Note that the subjects are those who carry both the hemoglobin A gene and the hemoglobin C gene, a mutant hemoglobin gene. As a result, chromatograms of hemoglobins shown in Figs. 1A to 1C are obtained. HbC is an example of mutant hemoglobin. In each diagram, the vertical axis of the chromatogram is absorbance detected from an optical detector which detects the concentration of hemoglobin, and the horizontal axis is time elapsed since the start of the measurement.

In the chromatogram of each diagram, the following peaks are observed in the order of a higher hemoglobin elution rate from the column. Specifically, a peak 12 (12a, 12b, and 12c correspond to the peak heights) containing hemoglobin A1a and hemoglobin A1b, a peak 14 (14a, 14b, and 14c corresponds to the peak heights) containing unstable hemoglobin A1c, a peak 16 (16a, 16b, and 16c corresponds to the peak heights) containing stable hemoglobin A1c, a peak 18 (18a, 18b, and 18c correspond to the peak heights) containing hemoglobin A0, a peak 20 (20a, 20b, and 20c correspond to the peak heights) described later, and a peak 22 (22a, 22b, and 22c correspond to the peak heights) containing HbC are observed in this order. In the following description, hemoglobin A1a is referred to as HbA1a, hemoglobin A1b is referred to as HbA1b, unstable hemoglobin A1c is referred to as L-A1c, stable hemoglobin A1c is referred to as HbA1c, and hemoglobin A0 is referred to as HbA0.

Here, the peak 20 is observed between the peak 18 of HbA0 and the peak 22 of HbC. The peak value of the peak 20 (for example, 20a, 20b, and 20c which are the peak heights) increases or decreases in accordance with the increase or decrease of the peak value of HbA1c (for example, 16a, 16b, and 16c which are the peak heights). Thus, the peak 20 can be determined as a peak containing a glycated product of HbC. In other words, the peak 20 is a peak containing glycated HbC. The peak 20 is an example of a peak X.

As described above, the peak value of glycated HbC (for example, 20a, 20b, and 20c which are the peak heights) is correlated with the peak value of HbA1c (for example, 16a, 16b, and 16c which are the peak heights). Note that a, b, and c attached to the end of the reference numerals may be omitted in a case where a, b, and c are not particularly distinguished.

Then, it is considered that there is a strong correlation between a value (hereinafter, referred to as a "glycated HbC level") based on the ratio of the peak value of the peak containing glycated HbC to the peak value of the total HbC (the total peak value of the peak value of the peak containing HbC and the peak value of the peak containing glycated HbC formed by glycation of HbC) and a value (hereinafter referred to as an "HbA1c level") based on the ratio of the peak value of the peak containing HbA1c to the peak value of the total HbA (the total peak value of the peak value of the peak containing HbA and the peak value of the peak containing modified HbA formed by modification of HbA).

The glycated HbC level is derived as glycated HbC% by the following Formula (1) when, for example, the sum of the peak area of glycated HbC (glycated HbC peak area) and the peak area of HbC (HbC peak area) is assumed to be the total area of HbC.

$$\text{Glycated HbC\% (glycated HbC level)} = \text{glycated HbC peak area/total area of HbC} \times 100 \quad \cdots$$

$$(1)$$

Note that the peak area of glycated HbC is the area within a range R20 in each diagram, and the peak area of HbC is the area within a range R22 in each diagram.

Meanwhile, the HbA1c level is derived as HbA1c% by the following Formula (2) when the sum of the peak area of HbA1a and HbA1b, the peak area of L-A1c, the peak area of HbA1c, and the peak area of HbA0 is assumed to be the total area of HbA (total value of the peak values of the peaks related to hemoglobin A).

$$\text{HbA1c\% (HbA1c level)} = \text{HbA1c peak area/total area of HbA} \times 100 \quad \cdots (2)$$

Note that the peak area of HbA1a and HbA1b (peak area of HbA1a + peak area of HbA1b) is the area within a range R12 in each diagram, the peak area of L-A1c is the area within a range R14 in each diagram, the peak area of HbA1c is the area within a range R16 in each diagram, and the peak area of HbA0 is the area within the range R18 in each diagram. In addition, the boundary of each range is defined by inflection points obtained by differentiating the curve of the chromatogram twice or points at which the absorbance shows the minimum value between each peak.

Since the peak 16 of HbA1c (16a, 16b, and 16c correspond to the peak heights) increases or decreases in accordance with the increase or decrease of the peak value of the peak 20 of glycated HbC, it is considered that there is a proportional relationship between the HbA1c level and the glycated HbC level. Then, it is considered that the following Correlation Formula (3) is established between the HbA1c level, which is an indicator of the blood glucose level, and the glycated HbC level in a blood specimen containing HbC.

$$\text{HbA1c level} = a_1 \times \text{glycated HbC level} + b_1 \cdots (3)$$

$a_1$: Slope (constant)
$b_1$: Intercept (constant)

In this manner, Correlation Formula (3) between the HbA1c level and the glycated HbC level is derived, which allows the HbA1c level, an indicator of the blood glucose level, to be estimated when the blood specimen is either heterozygous for HbC or homozygous for HbC without HbA, by substituting the glycated HbC level into Formula (3).

(Example)

Next, an example of the method for estimating an HbA1c level according to an embodiment of the present invention will be described using a total of 20 blood specimens containing HbA and HbC, each collected from a plurality of subjects.

(a) Liquid Chromatography Apparatus

A commercially available cation exchange chromatography column filled with a hydrophilic polymer formed of a methacrylic acid ester copolymer was connected to a commercially available high-performance liquid chromatography apparatus. An optical detector (specifically, an absorption spectrometer) for detecting the concentration of hemoglobin flowing through the flow path was attached to the cation exchange chromatography column at a predetermined position of the downstream flow path.

(b) Eluent

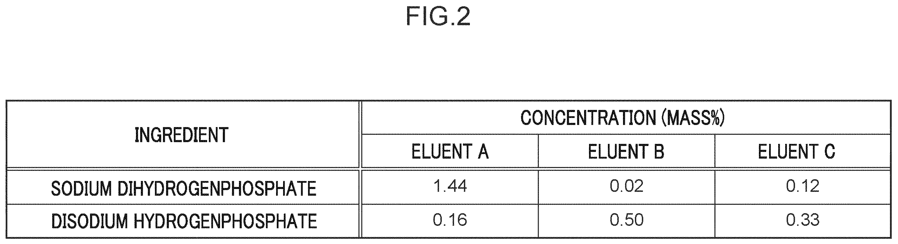

The aqueous solutions having the compositions indicated in the table shown in Fig. 2 were used as eluents A, B, and C. The pHs of the eluent A, eluent B, and eluent C were adjusted to pH 5.08, pH 8.0, and pH 6.82, respectively. the hemoglobin elution power of the eluent was the lowest in eluent A and the highest in eluent B.

(c) Chromatogram

The column was equilibrated by allowing the eluent A to flow through the liquid chromatography apparatus of (a) above. Then, a predetermined amount of a diluted blood specimen was introduced into the column. Next, the eluent A was allowed to flow for a predetermined amount of time (for example, 13 seconds), whereby HbF or HbA1c was eluted. Next, a solution obtained by mixing the eluent A and the eluent C at a predetermined ratio (for example, 1:9) was allowed to flow for a predetermined amount of time (for example, 5 seconds), and then the eluent C was allowed to flow for a predetermined amount of time (for example, 17 seconds), whereby HbA0 was eluted. Next, all hemoglobin remaining in the analytical column was eluted by allowing the eluent B to flow for a predetermined amount of time (for example, 2 seconds). Then, the eluent A was allowed to flow for a predetermined amount of time (for example, 5 seconds). During the elution, a chromatogram was created using the absorbance obtained from the optical detector at a detection wavelength of 420 nm. As a result, a total of 10 chromatograms were obtained from 10 blood specimens containing HbA and HbC each collected from the plurality of subjects.

In the obtained chromatograms, the peak values of the peaks 20 eluted before the peaks 22 of HbC increased or decreased in accordance with the increase or decrease of the peak values of the peaks 16 of HbA1c.

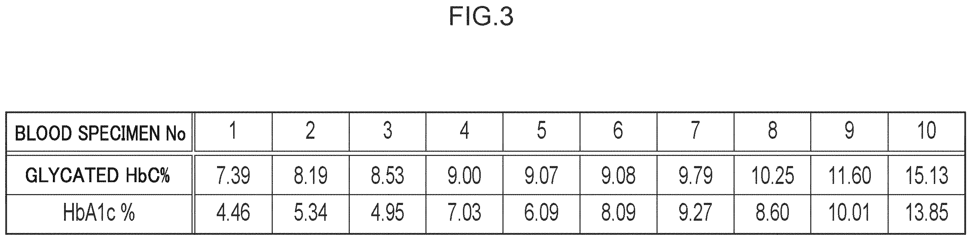

The glycated HbC% derived from each chromatogram using Formula (1) described above and the HbA1c% derived using Formula (2) are indicated in the table shown in Fig. 3.

In addition, for the glycated HbC% and the HbA1c% shown in Fig. 3, $a_1$ and $b_1$ of Correlation Formula (3) were obtained. As a result, $a_1$ was 1.2365, and $b_1$ was -4.3522. That is, the following Correlation Formula (4) was obtained using the 10 blood specimens.

$$\text{HbA1c\%} = 1.2365 \times \text{glycated HbC\%} - 4.3522 \cdots (4)$$

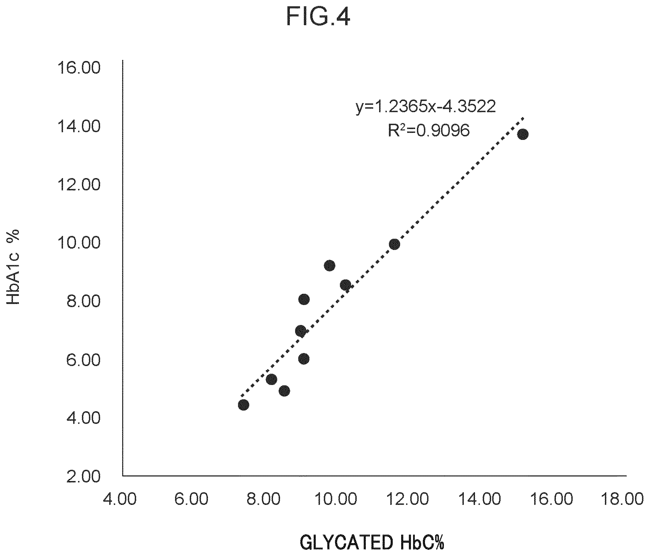

Furthermore, the graph in Fig. 4 shows the points obtained by plotting the values of the HbA1c% against the values of the glycated HbC% that are indicated in the table of Fig. 3 and the regression line representing Correlation Formula (4). The vertical axis of the graph shown in Fig. 4 is the HbA1c%, and the horizontal axis is the glycated HbC%. The coefficient of determination ($R^2$) of the regression line is 0.9096, which is a high value close to 1, and thus, it is understood that there is a strong correlation between the glycated HbC% and the HbA1c%.

(d) Validation

Next, Correlation Formula (4) is validated. The validation was performed using 10 new blood specimens containing HbA and HbC, each collected from a plurality of subjects. Regarding the 10 new blood specimens, the specimens are different from the 10 blood specimens used for deriving Correlation Formula (4) described above, and the HbA1c levels therein are known.

Using these 10 new blood specimens, the glycated HbC% and the HbA1c% (measured values) were derived by the same methods as (a), (b), and (c) described above. Furthermore, the derived glycated HbC% was substituted into Correlation Formula (4), thereby deriving the estimated values of the HbA1c%.

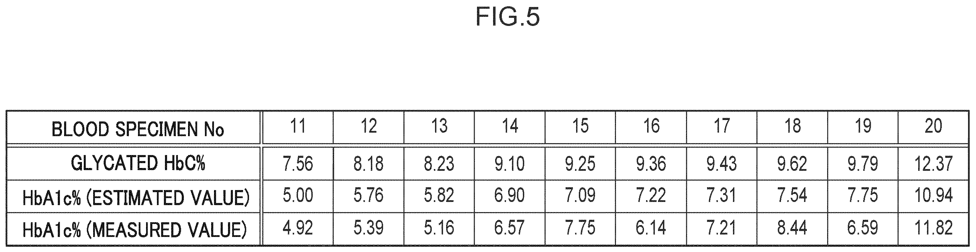

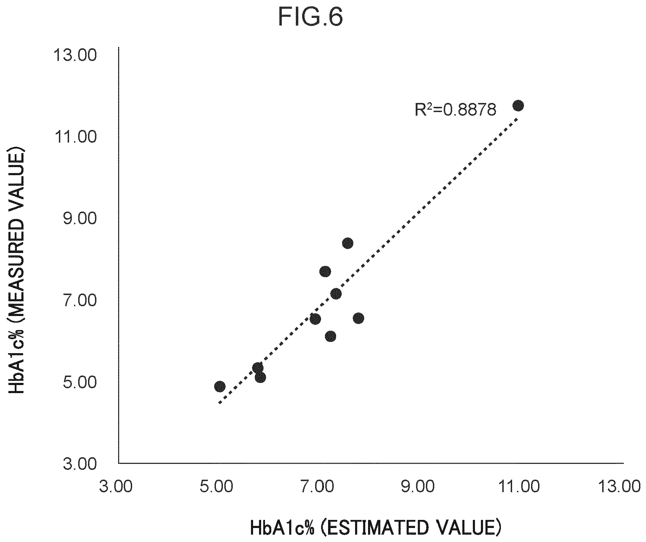

The glycated HbC% obtained from the chromatogram and the HbA1c% (measured values) and the HbA1c% obtained from Correlation Formula (4) (estimated values) are indicated in the table shown in Fig. 5. Furthermore, the graph shown in Fig. 6 shows the regression line representing the correlation between the HbA1c% obtained from the correlation formula (estimated value) and the HbA1c% (measured value) and the points obtained by plotting the measured values of the HbA1c% against the estimated values of the HbA1c% obtained from the correlation formula, which are shown in the table of Fig. 5. The vertical axis of the graph shown in Fig. 6 is the HbA1c% (measured values), and the horizontal axis is the HbA1c% (estimated values). The coefficient of determination ($R^2$) of the regression line is 0.8878, which is a high value close to 1, and thus, it is understood that, by using Correlation Formula (4), the HbA1c% can be estimated from the glycated HbC% derived from a chromatogram.

(Information Processing Apparatus 100)

Next, an information processing apparatus 100 will be described, which executes a program for estimating the HbA1c level using the method for estimating an HbA1c level described above.

Hardware Configuration of Information Processing Apparatus 100

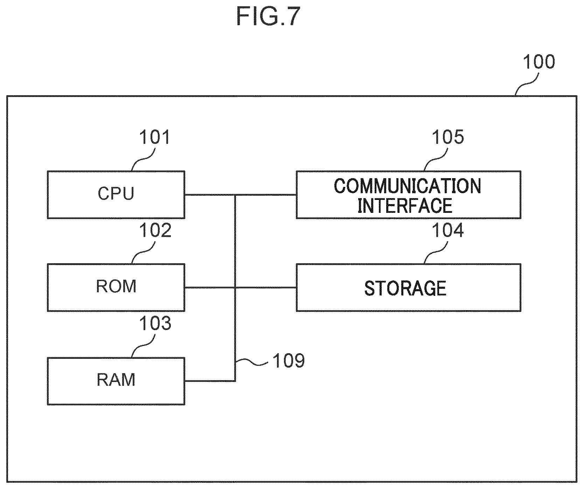

As shown in Fig. 7, the information processing apparatus 100 includes a central processing unit (CPU) 101, a read only memory (ROM) 102, a random access memory (RAM) 103, a storage 104, and a communication interface (I/F) 105. The respective components are connected to each other via a bus 109 so as to be able to communicate with each other.

The CPU 101 is a central processing unit which executes various programs or controls each part. In other words, the CPU 101 reads a program from the ROM 102 or the storage 104 and executes the program using the RAM 103 as the work area. The CPU 101 controls each component and performs various calculation processes according to the program stored in the ROM 102 or the storage 104.

The ROM 102 stores various programs and various data. The RAM 103 is a work area which temporarily stores a program or data. The storage 104 is configured of a hard disk drive (HDD) or a solid state drive (SSD) and stores various programs including an operating system and various data. The communication interface 105 is an interface for the communication between the information processing apparatus 100 and the liquid chromatography apparatus or the like. For example, standards such as Ethernet (registered trademark), FDDI, Wi-Fi (registered trademark), and the like are used.

When executing the program for estimating the HbA1c level, the information processing apparatus 100 uses the above-described hardware resources to realize various functions.

Functional Configuration of Information Processing Apparatus 100

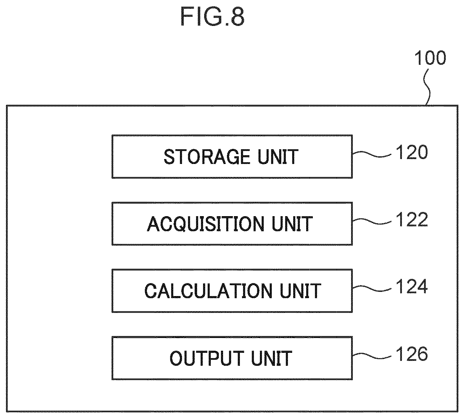

As shown in Fig. 8, the information processing apparatus 100 includes a storage unit 120, an acquisition unit 122, a calculation unit 124, and an output unit 126. Each functional configuration is realized by the CPU 101 reading and executing the estimation program stored in the ROM 102 or the storage 104.

The storage unit 120 stores Correlation Formula (3) described above that is a correlation between the HbA1c level, which is the indicator of the blood glucose level, and the glycated HbC level. That is, Correlation Formula (3) is stored in the storage unit 120 of the information processing apparatus 100 in advance.

The acquisition unit 122 acquires the glycated HbC level obtained from a chromatogram that is obtained by subjecting a blood specimen which is a measurement target collected from a subject having HbC to liquid chromatography. As the glycated hemoglobin C level, a value may be acquired which is based on the ratio of the peak value of a peak X that appears between the peak containing hemoglobin A0 and the hemoglobin C peak containing hemoglobin C to the total value of the peak value of the peak X and the peak value of the hemoglobin C peak, the peaks being obtained in a chromatogram that is obtained by subjecting the blood specimen which is the measurement target collected from the subject having HbC to liquid chromatography.

The calculation unit 124 calculates the estimated value of the HbA1c level by substituting the glycated HbC level acquired by the acquisition unit 122 into Correlation Formula (3) stored in the storage unit 120.

The output unit 126 outputs the estimated value of the HbA1c level calculated by the calculation unit 124 to a display (not shown).

Operation of Information Processing Apparatus 100

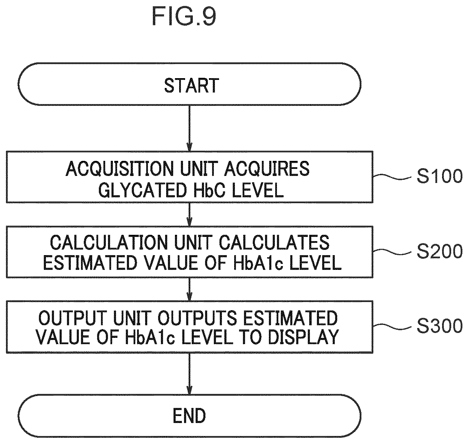

Next, the operation of the information processing apparatus 100 will be described using the flow diagram shown in Fig. 9.

In a step S100 shown in Fig. 9, the acquisition unit 122 in the information processing apparatus 100 which stores Correlation Formula (3) in the storage unit 120 in advance acquires the glycated HbC level obtained from a chromatogram that is obtained by subjecting a blood specimen which is a measurement target collected from a subject having HbC to liquid chromatography.

Furthermore, in a step S200, the calculation unit 124 calculates the estimated value of the HbA1c level by substituting the glycated HbC level acquired by the acquisition unit 122 into Correlation Formula (3) stored in the storage unit 120.

In addition, in a step S300, the output unit 126 outputs the estimated value of the HbA1c level calculated by the calculation unit 124 to a display (not shown). Thus, a series of steps are completed.

(Summary)

As described above, by acquiring in advance Correlation Formula (3) between the HbA1c level and the glycated HbC level from chromatograms that are obtained by subjecting a plurality of blood specimens containing HbA and HbC to liquid chromatography, the HbA1c level which is an indicator of the blood glucose level can be estimated even with a blood specimen containing HbC.

Although the HbA1c level, which is an indicator of the blood glucose level, has been estimated in the above embodiment, directly measuring the blood glucose level is also considered. However, since the blood glucose level fluctuates according to the physical condition, estimating the HbA1c level suppresses the fluctuation which occurs due to the physical condition.

Although specific embodiments of the present invention have been described in detail, the present invention is not limited to such embodiments, and various other embodiments can be adopted within the scope of the present invention, which is clear to those skilled in the art. Although description has been made in the above embodiment using HbC as the mutant hemoglobin, the mutant hemoglobin may also be, for example, hemoglobin S, hemoglobin E, or hemoglobin D.

Furthermore, in the above embodiment, the HbA1c level which is an indicator of the blood glucose level has been estimated from the glycated mutant hemoglobin level that is derived by analyzing a heterozygote of HbA and the mutant hemoglobin and Correlation Formula (3). However, the HbA1c level which is an indicator of the blood glucose level may also be estimated from a glycated mutant hemoglobin level that is derived by analyzing a homozygote of the mutant hemoglobin and the mutant hemoglobin and Correlation Formula (3). In other words, the HbA1c level which is an indicator of the blood glucose level may also be estimated with a blood specimen not containing HbA.

Moreover, while the total area of HbC has been used as the denominator in order to derive the glycated HbC level in the above embodiment, the total peak area of the chromatogram may also be used as the denominator.

**Claims**

1.  A method for estimating a hemoglobin A1c level, the method comprising:

    acquiring in advance a correlation between a hemoglobin A1c level which is a value based on the ratio of the peak value of a hemoglobin A1c peak containing hemoglobin A1c to the total value of the peak values of peaks related to hemoglobin A and a glycated mutant hemoglobin level which is a value based on the ratio of the peak value of a glycated mutant hemoglobin peak to the total value of the peak value of a peak containing mutant hemoglobin and the peak value of the glycated mutant hemoglobin peak containing glycated mutant hemoglobin formed by glycating the mutant hemoglobin, from a chromatogram obtained by subjecting a blood specimen containing the hemoglobin A and the mutant hemoglobin that is collected from each of a plurality of subjects to liquid chromatography;
    deriving a glycated mutant hemoglobin level from a chromatogram obtained by subjecting a blood specimen which is a measurement target collected from a subject having the mutant hemoglobin to liquid chromatography; and
    estimating a hemoglobin A1c level based on the derived glycated mutant hemoglobin level and the correlation.

2.  The method for estimating a hemoglobin A1c level according to claim 1, wherein
    the mutant hemoglobin is hemoglobin C.

3.  The method for estimating a hemoglobin A1c level according to claim 2, wherein
    a glycated hemoglobin C level is obtained as a value based on the ratio of the peak value of a peak X, which appears between a peak containing hemoglobin A0 and a hemoglobin C peak containing hemoglobin C in the chromatogram, to the total value of the peak value of the peak X and the peak value of the hemoglobin C peak and used as the glycated mutant hemoglobin level.

4.  The method for estimating a hemoglobin A1c level according to any of claims 1 to 3, wherein
    the liquid chromatography is cation exchange chromatography.

5.  An information processing apparatus comprising:

    a storage unit (120) configured to store a correlation between a hemoglobin A1c level which is a value based on the ratio of the peak value of a hemoglobin A1c peak containing hemoglobin A1c to the total value of the peak values of peaks related to hemoglobin A and a glycated mutant hemoglobin level which is a value based on the ratio of the peak value of a glycated mutant hemoglobin peak to the total value of the peak value of a peak containing mutant hemoglobin and the peak value of the glycated mutant hemoglobin peak containing glycated mutant hemoglobin formed by glycating the mutant hemoglobin, the correlation being obtained from a chromatogram obtained by subjecting a blood specimen containing the hemoglobin A and the mutant hemoglobin that is collected from each of a plurality of subjects to liquid chromatography;
    an acquisition unit (122) configured to acquire a glycated mutant hemoglobin level obtained from a chromatogram obtained by subjecting a blood specimen which is a measurement target collected from a subject having the mutant hemoglobin to liquid chromatography;
    a calculation unit (124) configured to calculate an estimated value of a hemoglobin A1c level based on the glycated mutant hemoglobin level acquired by the acquisition unit and the correlation stored in the storage unit; and

an output unit (126) configured to output the estimated value.

6. An estimation computer program product which causes a computer to store a correlation between a hemoglobin A1c level which is a value based on the ratio of the peak value of a hemoglobin A1c peak containing hemoglobin A1c to the total value of the peak values of peaks related to hemoglobin A and a glycated mutant hemoglobin level which is a value based on the ratio of the peak value of a glycated mutant hemoglobin peak to the total value of the peak value of a peak containing mutant hemoglobin and the peak value of the glycated mutant hemoglobin peak containing glycated mutant hemoglobin formed by glycating the mutant hemoglobin, the correlation being obtained from a chromatogram obtained by subjecting a blood specimen containing the hemoglobin A and the mutant hemoglobin that is collected from each of a plurality of subjects to liquid chromatography, and to execute a method comprising the steps of:

acquiring a glycated mutant hemoglobin level obtained from a chromatogram obtained by subjecting a blood specimen which is a measurement target collected from a subject having the mutant hemoglobin to liquid chromatography,
calculating an estimated value of a hemoglobin A1c level based on the acquired glycated mutant hemoglobin level and the stored correlation, and
outputting the estimated value.

7. A non-transitory computer-readable storage medium storing the estimation computer program product according to claim 6.

**Patentansprüche**

1. Verfahren zum Schätzen des Hämoglobin-A1c-Spiegels, wobei das Verfahren umfasst:

Vorabermitteln einer Korrelation zwischen einem Hämoglobin-A1c-Spiegel, der ein Wert basierend auf dem Verhältnis des Spitzenwerts einer Hämoglobin-A1c-Spitze, die Hämoglobin A1c enthält, zum Gesamtwert der Spitzenwerte von Spitzen, die sich auf Hämoglobin A beziehen, und einem Spiegel an glykiertem mutiertem Hämoglobin, der ein Wert basierend auf dem Verhältnis des Spitzenwerts einer Spitze an glykiertem mutiertem Hämoglobin zum Gesamtwert des Spitzenwerts einer Spitze, die mutiertes Hämoglobin enthält, und dem Spitzenwert der Spitze an glykiertem mutiertem Hämoglobin, die glykiertes mutiertes Hämoglobin enthält, das durch Glykierung des mutierten Hämoglobins gebildet wurde, ist, aus einem Chromatogramm, das durch Unterziehen einer Blutprobe, die das Hämoglobin A und das mutierte Hämoglobin enthält, die von jedem einer Vielzahl von Subjekten entnommen wurde, einer Flüssigchromatographie erhalten wird;
Ableiten des glykierten mutierten Hämoglobinspiegels aus einem Chromatogramm, das durch Unterziehen einer Blutprobe, die ein Messobjekt ist, das von einem Subjekt entnommen wurde, welches das mutierte Hämoglobin aufweist, einer Flüssigchromatographie erhalten wird; und
Schätzen eines Hämoglobin-A1c-Spiegels basierend auf dem abgeleiteten glykierten mutierten Hämoglobinspiegel und der Korrelation.

2. Verfahren zum Schätzen des Hämoglobin-A1c-Spiegels nach Anspruch 1, wobei das mutierte Hämoglobin Hämoglobin C ist.

3. Verfahren zum Schätzen des Hämoglobin-A1c-Spiegels nach Anspruch 2, wobei ein glykierter Hämoglobin-C-Spiegel als Wert basierend auf dem Verhältnis des Spitzenwerts einer Spitze X, die zwischen einer Spitze, die Hämoglobin A0 enthält, und einer Hämoglobin-C-Spitze, die Hämoglobin C enthält, im Chromatogramm erscheint, zum Gesamtwert des Spitzenwerts der Spitze X und des Spitzenwerts der Hämoglobin-C-Spitze erhalten wird, und als glykierter mutierter Hämoglobinspiegel verwendet wird.

4. Verfahren zum Schätzen des Hämoglobin-A1c-Spiegels nach einem der Ansprüche 1 bis 3, wobei die Flüssigchromatographie eine Kationenaustauschchromatographie ist.

5. Informationsverarbeitungseinrichtung, umfassend:

eine Speichereinheit (120), die konfiguriert ist, um eine Korrelation zwischen einem Hämoglobin-A1c-Spiegel, der ein Wert basierend auf dem Verhältnis des Spitzenwerts einer Hämoglobin-A1c-Spitze, die Hämoglobin A1c enthält, zum Gesamtwert der Spitzenwerte von Spitzen, die sich auf Hämoglobin A beziehen, und einem Spiegel an glykiertem mutiertem Hämoglobin, der ein Wert basierend auf dem Verhältnis des Spitzenwerts einer Spitze an glykiertem mutiertem Hämoglobin zum Gesamtwert des Spitzenwerts einer Spitze, die mutiertes Hämoglobin enthält, und dem Spitzenwert der Spitze an glykiertem mutiertem Hämoglobin, die glykiertes mutiertes Hämoglobin enthält, das durch Glykierung des mutierten Hämoglobins gebildet wurde, zu speichern, wobei die Korrelation aus einem Chromatogramm, das durch Unterziehen einer Blutprobe, die das Hämoglobin A und das mutierte Hämoglobin enthält, die von jedem einer Vielzahl von Subjekten entnommen wurde, einer Flüssigchromatographie erhalten wird;

eine Ermittlungseinheit (122), die konfiguriert ist, um einen glykierten mutierten Hämoglobinspiegel aus einem Chromatogramm zu ermitteln, das durch Unterziehen einer Blutprobe, die ein Messobjekt ist, das von einem Subjekt entnommen wurde, welches das mutierte Hämoglobin aufweist, einer Flüssigchromatographie erhalten wird;

eine Berechnungseinheit (124), die konfiguriert ist, um einen geschätzten Wert eines Hämoglobin-A1c-Spiegels basierend auf dem von der Ermittlungseinheit ermittelten glykierten mutierten Hämoglobinspiegel und der in der Speichereinheit gespeicherten Korrelation zu berechnen; und

eine Ausgabeeinheit (126), die konfiguriert ist, um den geschätzten Wert auszugeben.

6. Computerprogrammprodukt zum Schätzen, das einen Computer veranlasst, eine Korrelation zwischen einem Hämoglobin-A1c-Spiegel, der ein Wert basierend auf dem Verhältnis des Spitzenwerts einer Hämoglobin-A1c-Spitze, die Hämoglobin A1c enthält, zum Gesamtwert der Spitzenwerte von Spitzen, die sich auf Hämoglobin A beziehen, und einem Spiegel an glykiertem mutiertem Hämoglobin, der ein Wert basierend auf dem Verhältnis des Spitzenwerts einer Spitze an glykiertem mutiertem Hämoglobin zum Gesamtwert des Spitzenwerts einer Spitze, die mutiertes Hämoglobin enthält, und dem Spitzenwert der Spitze an glykiertem mutiertem Hämoglobin, die glykiertes mutiertes Hämoglobin enthält, das durch Glykierung des mutierten Hämoglobins gebildet wurde, zu speichern, wobei die Korrelation aus einem Chromatogramm, das durch Unterziehen einer Blutprobe, die das Hämoglobin A und das mutierte Hämoglobin enthält, die von jedem einer Vielzahl von Subjekten entnommen wurde, einer Flüssigchromatographie erhalten wird, und ein Verfahren auszuführen, das die Schritte umfasst zum:

Ermitteln eines glykierten mutierten Hämoglobinspiegels, der aus einem Chromatogramm erhalten wird, das durch Unterziehen einer Blutprobe, die ein Messobjekt ist, das von einem Subjekt entnommen wurde, welches das mutierte Hämoglobin aufweist, einer Flüssigchromatographie erhalten wird,

Berechnen eines geschätzten Wertes eines Hämoglobin-A1c-Spiegels basierend auf dem ermittelten glykierten mutierten Hämoglobinspiegel und der gespeicherten Korrelation, und

Ausgeben des geschätzten Wertes.

7. Nichtflüchtiges, computerlesbares Speichermedium, welches das Computerprogrammprodukt zum Schätzen nach Anspruch 6 speichert.

## Revendications

1. Procédé d'estimation d'un taux d'hémoglobine A1c, le procédé comprenant les étapes consistant à :

acquérir à l'avance une corrélation entre un taux d'hémoglobine A1c, qui est une valeur basée sur le rapport de la valeur de pic d'un pic d'hémoglobine A1c contenant de l'hémoglobine A1c à la valeur totale des valeurs de pic de pics liés à l'hémoglobine A, et un taux d'hémoglobine mutante glyquée, qui est une valeur basée sur le rapport de la valeur de pic d'un pic d'hémoglobine mutante glyquée à la valeur totale de la valeur de pic d'un pic contenant de l'hémoglobine mutante et la valeur de pic du pic d'hémoglobine mutante glyquée contenant de l'hémoglobine mutante glyquée formée par glycation de l'hémoglobine mutante, à partir d'un chromatogramme obtenu en soumettant un échantillon de sang contenant de l'hémoglobine A et de l'hémoglobine mutante qui est collecté auprès de chacun d'une pluralité de sujets à une chromatographie liquide ;

déduire un taux d'hémoglobine mutante glyquée à partir d'un chromatogramme obtenu en soumettant un échantillon de sang qui est une cible de mesure collectée auprès d'un sujet présentant l'hémoglobine mutante à une chromatographie liquide ; et

estimer un taux d'hémoglobine A1c sur la base du taux d'hémoglobine mutante glyquée déduit et de la corrélation.

2. Procédé d'estimation d'un taux d'hémoglobine A1c selon la revendication 1, dans lequel l'hémoglobine mutante est de l'hémoglobine C.

3. Procédé d'estimation d'un taux d'hémoglobine A1c selon la revendication 2, dans lequel
un taux d'hémoglobine C glyquée est obtenu sous la forme d'une valeur basée sur le rapport de la valeur de pic d'un pic X, qui apparaît entre un pic contenant de l'hémoglobine A0 et un pic d'hémoglobine C contenant de l'hémoglobine C dans le chromatogramme, à la valeur totale de la valeur de pic du pic X et de la valeur de pic du pic d'hémoglobine C, et utilisée comme taux d'hémoglobine mutante glyquée.

4. Procédé d'estimation d'un taux d'hémoglobine A1c selon l'une quelconque des revendications 1 à 3, dans lequel la chromatographie liquide est une chromatographie par échange de cations.

5. Appareil de traitement d'informations, comprenant :

une unité de stockage (120) configurée pour stocker une corrélation entre un taux d'hémoglobine A1c, qui est une valeur basée sur le rapport de la valeur de pic d'un pic d'hémoglobine A1c contenant de l'hémoglobine A1c à la valeur totale des valeurs de pic de pics liés à l'hémoglobine A, et un taux d'hémoglobine mutante glyquée, qui est une valeur basée sur le rapport de la valeur de pic d'un pic d'hémoglobine mutante glyquée à la valeur totale de la valeur de pic d'un pic contenant de l'hémoglobine mutante et la valeur de pic du pic d'hémoglobine mutante glyquée contenant de l'hémoglobine mutante glyquée formée par glycation de l'hémoglobine mutante, la corrélation étant obtenue à partir d'un chromatogramme obtenu en soumettant un échantillon de sang contenant de l'hémoglobine A et de l'hémoglobine mutante qui est collecté auprès de chacun d'une pluralité de sujets à une chromatographie liquide ;
une unité d'acquisition (122) configurée pour acquérir un taux d'hémoglobine mutante glyquée obtenu à partir d'un chromatogramme obtenu en soumettant un échantillon de sang qui est une cible de mesure collectée auprès d'un sujet présentant l'hémoglobine mutante à une chromatographie liquide ;
une unité de calcul (124) configurée pour calculer une valeur estimée d'un taux d'hémoglobine A1c sur la base du taux d'hémoglobine mutante glyquée acquis par l'unité d'acquisition et de la corrélation stockée dans l'unité de stockage ; et
une unité de sortie (126) configurée pour délivrer en sortie la valeur estimée.

6. Produit de programme informatique d'estimation qui amène un ordinateur à stocker une corrélation entre un taux d'hémoglobine A1c, qui est une valeur basée sur le rapport de la valeur de pic d'un pic d'hémoglobine A1c contenant de l'hémoglobine A1c à la valeur totale des valeurs de pic de pics liés à l'hémoglobine A, et un taux d'hémoglobine mutante glyquée, qui est une valeur basée sur le rapport de la valeur de pic d'un pic d'hémoglobine mutante glyquée à la valeur totale de la valeur de pic d'un pic contenant de l'hémoglobine mutante et la valeur de pic du pic d'hémoglobine mutante glyquée contenant de l'hémoglobine mutante glyquée formée par glycation de l'hémoglobine mutante, la corrélation étant obtenue à partir d'un chromatogramme obtenu en soumettant un échantillon de sang contenant de l'hémoglobine A et de l'hémoglobine mutante qui est collecté auprès de chacun d'une pluralité de sujets à une chromatographie liquide, et à exécuter un procédé comprenant les étapes consistant à :

acquérir un taux d'hémoglobine mutante glyquée obtenu à partir d'un chromatogramme obtenu en soumettant un échantillon de sang qui est une cible de mesure collectée auprès d'un sujet présentant l'hémoglobine mutante à une chromatographie liquide,
calculer une valeur estimée d'un taux d'hémoglobine A1c sur la base du taux d'hémoglobine mutante glyquée acquis et de la corrélation stockée, et
délivrer en sortie la valeur estimée.

7. Support de stockage non transitoire lisible par ordinateur stockant le produit de programme informatique d'estimation selon la revendication 6.

FIG.1A

FIG.1B

EP 4 386 001 B1

FIG.1C

EP 4 386 001 B1

## FIG.2

| INGREDIENT | CONCENTRATION (MASS%) | | |
|---|---|---|---|
| | ELUENT A | ELUENT B | ELUENT C |
| SODIUM DIHYDROGENPHOSPHATE | 1.44 | 0.02 | 0.12 |
| DISODIUM HYDROGENPHOSPHATE | 0.16 | 0.50 | 0.33 |

FIG.3

| BLOOD SPECIMEN No | 1 | 2 | 3 | 4 | 5 | 6 | 7 | 8 | 9 | 10 |
|---|---|---|---|---|---|---|---|---|---|---|
| GLYCATED HbC% | 7.39 | 8.19 | 8.53 | 9.00 | 9.07 | 9.08 | 9.79 | 10.25 | 11.60 | 15.13 |
| HbA1c % | 4.46 | 5.34 | 4.95 | 7.03 | 6.09 | 8.09 | 9.27 | 8.60 | 10.01 | 13.85 |

## FIG.4

$y=1.2365x-4.3522$
$R^2=0.9096$

HbA1c %

GLYCATED HbC%

FIG.5

| BLOOD SPECIMEN No | 11 | 12 | 13 | 14 | 15 | 16 | 17 | 18 | 19 | 20 |
|---|---|---|---|---|---|---|---|---|---|---|
| GLYCATED HbC% | 7.56 | 8.18 | 8.23 | 9.10 | 9.25 | 9.36 | 9.43 | 9.62 | 9.79 | 12.37 |
| HbA1c% (ESTIMATED VALUE) | 5.00 | 5.76 | 5.82 | 6.90 | 7.09 | 7.22 | 7.31 | 7.54 | 7.75 | 10.94 |
| HbA1c% (MEASURED VALUE) | 4.92 | 5.39 | 5.16 | 6.57 | 7.75 | 6.14 | 7.21 | 8.44 | 6.59 | 11.82 |

FIG.6

FIG.7

100

101

CPU

105

COMMUNICATION
INTERFACE

102

ROM

104

STORAGE

103

RAM

109

FIG.8

100

| | |
|---|---|
| STORAGE UNIT | 120 |
| ACQUISITION UNIT | 122 |
| CALCULATION UNIT | 124 |
| OUTPUT UNIT | 126 |

# FIG.9

START

ACQUISITION UNIT ACQUIRES
GLYCATED HbC LEVEL — S100

CALCULATION UNIT CALCULATES
ESTIMATED VALUE OF HbA1c LEVEL — S200

OUTPUT UNIT OUTPUTS ESTIMATED
VALUE OF HbA1c LEVEL TO DISPLAY — S300

END

**EP 4 386 001 B1**

**Patent documents cited in the description**

- JP 2012215470 A